Europäisches Patentamt

European Patent Office

Office européen des brevets

(19)

(11) Veröffentlichungsnummer: **0 149 425**
A1

(12) # EUROPÄISCHE PATENTANMELDUNG

(21) Anmeldenummer: **84810616.7**

(22) Anmeldetag: **14.12.84**

(51) Int. Cl.⁴: **A 61 F 2/34**
A 61 L 27/00, A 61 F 2/30

(30) Priorität: **11.01.84 CH 120/84**

(43) Veröffentlichungstag der Anmeldung:
**24.07.85 Patentblatt 85/30**

(84) Benannte Vertragsstaaten:
**AT BE DE FR GB**

(71) Anmelder: **Robert Mathys Co**
**Güterstrasse 5**
**CH-2544 Bettlach(CH)**

(72) Erfinder: **Robert Mathys Co**
**Güterstrasse 5**
**CH-2544 Bettlach(CH)**

(74) Vertreter: **Eder, Carl E. et al,**
**Patentanwaltsbüro Eder & Cie Münchensteinerstrasse 2**
**CH-4052 Basel(CH)**

(54) **Aus Kunststoff bestehende Gelenkpfanne.**

(57) Die aus Kunststoff, vorzugsweise Polyäthylen. bestehende Gelenkpfanne ist mit einer Schicht aus gesintertem Hydroxylapatit überzogen, die vorzugsweise die Dicke eines Hydroxylapatitkorns aufweist.

Fig. 1

EP 0 149 425 A1

Robert Mathys Co., Bettlach (Schweiz)

## Aus Kunststoff bestehende Gelenkpfanne

Die vorliegende Erfindung betrifft eine aus Kunststoff bestehende Gelenkpfanne, die als Ersatz für eine natürliche Gelenkpfanne bei Mensch und Tier dienen kann und die zum zementfreien Einsetzen in den Knochen bestimmt ist. Die bekannten Gelenkpfannen dieser Art weisen eine gerippte oder geriffelte oder evt. anderswie gerauhte Aussenfläche auf, damit der Knochen die Möglichkeit hat, sich beim Wachsen in den Unebenheiten der Aussenfläche einer solchen Prothese zu verankern.

Die Gelenkpfanne nach der vorliegenden Erfindung ist nur dadurch gekennzeichnet, dass sie auf der mit dem Knochen in Kontakt zu bringenden Aussenseite mindestens poröse Hydroxylapatit-Körner trägt. Diese Körner können eine Grösse von 100 - 200 um aufweisen und so dicht beieinander liegen, dass sie eine poröse Schicht bilden, die die Dicke eines Kornes aufweist und die sich mindestens über einen Teil der Aussenfläche erstreckt. Nachfolgend wird anhand der beiliegenden Zeichnung ein Ausführungsbeispiel der Erfindung beschrieben.

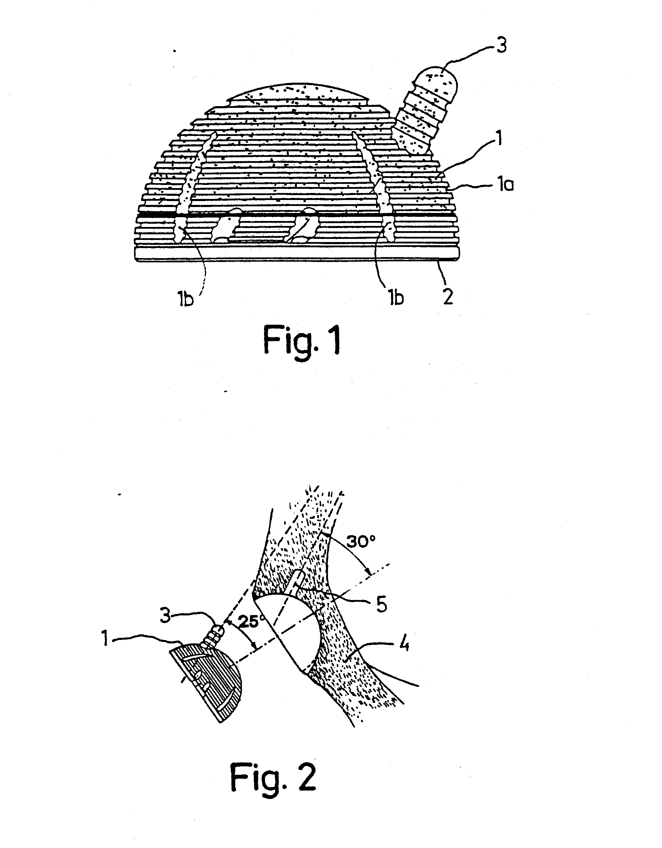

Die Figur 1     zeigt eine Seitenansicht einer erfindungsgemässen Gelenkpfanne und zwar einer Hüftgelenkpfanne,

die Figur 2     zeigt dieselbe Gelenkpfanne von der anderen Seite in kleinerem Massstab zusammen mit einem Schnitt durch den

16539/Ed/sr/Fall 12

für das Einsetzten vorbereiteten Knochen.

Es handelt sich um eine im wesentlichen halbkugelförmige Kalotte, deren in der Zeichnung nicht sichtbare Innenfläche hochglanzpoliert ist und deren Aussenfläche 1 mit Rillen 1a versehen ist, die zur Öffnungsebene 2 parallel verlaufen.

Zusätzlich sind noch einige Rillen 1b vorhanden, von denen in der Zeichnung zwei sichtbar sind und die ungefähr senkrecht zu den Rillen 1a verlaufen. Alle diese Rillen dienen dazu, dass der Knochen sich beim Wachsen an der Prothese verankern kann. Zwei Zapfen 3, von denen jedoch in der Zeichnung nur einer sichtbar ist und die ebenfalls mit Rillen versehen sind, stehen schief auf der Aussenfläche der Gelenkpfanne. Sie werden in entsprechende, am Knochen 4 anzubringende Bohrlöcher 5 eingesteckt, wobei darauf zu achten ist, dass der Winkel zwischen den Bohrlöchern 5 und den Zapfen um ca. 5° differiert, damit die Zapfen 3 beim Einsetzen verbogen werden, was ihnen die nötige Vorspannung gibt, um die Gelenkpfanne fest an der eingesetzten Stelle zu halten. Damit sich nun die Knochensubstanz beim Anwachsen noch besser als bisher üblich mit der Prothese verbinden kann, ist ihre Aussenfläche, wenigstens teilweise, mit einer in der Zeichnung punktiert gezeichneten Schicht aus gesintertem Hydroxylapatit überzogen. Diese Schicht braucht nicht dick zu sein, es genügt, wenn sie die Dicke eines einzelnen Kornes aufweist. In diesem Fall sitzt praktisch jenes Korn, das üblicherweise einen Durchmesser von 100 - 200 um hat und porös ist, einzeln auf der Kunststoffoberfläche. Versuche haben gezeigt, dass eine mit einer derartigen Schicht versehene Oberfläche ein bedeutend besseres und rascheres An- und Einwachsen des Knochens bewirkt als die bisher bekannten, durch Rillen oder andere Massnahmen strukturierten Kunststoff-Oberflächen.

Das Aufbringen der Hydroxylapatit-Körner auf die Kunststoffprothese, die vorzugsweise aus Polyäthylen bestehen kann, kann auf an sich bekannte Art erfolgen, beispielsweise durch Plasmaspray oder durch Aufsinterung.

**0149425**

<u>PATENTANSPRÜCHE</u>

1. Als Ersatz für eine natürliche Gelenkpfanne bei Mensch und Tier dienende, aus einem Kunststoff bestehende, zum zementfreien Einsetzen in den Knochen bestimmte Gelenkpfanne, dadurch gekennzeichnet, dass sie auf ihrer mit dem Knochen in Kontakt zu bringenden Aussenseite poröse Hydroxylapatit-Körner trägt.

2. Gelenkpfanne nach Anspruch 1, dadurch gekennzeichnet, dass die Hydroxylapatit-Körner gesintert sind.

3. Gelenkpfanne nach Anspruch 1 oder 2, dadurch gekennzeichnet, dass die Körner eine Grösse von 100 bis 200 um aufweisen.

4. Gelenkpfanne nach einem der Ansprüche 1 bis 3, dadurch gekennzeichnet, dass die Körner so dicht beieinander liegen, dass sie eine poröse Schicht bilden, die die Dicke eines Kornes aufweist.

Fig. 1

Fig. 2

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| Y | US-A-3 808 606 (TRONZO) <br> * Anspruch 1; Figuren 2-5 * | 1 | A 61 F 2/34 <br> A 61 L 27/00 <br> A 61 F 2/30 |
| Y | DE-A-2 008 010 (SCHNEIDER & CO.) <br> * Anspruch 1 * | 1 | |
| A | US-A-3 986 212 (SAUER) <br> * Spalte 4, Zeilen 1-15; Figur 2 * | 1 | |
| A | US-A-3 840 904 (TRONZO) <br> * Spalte 2, Zeilen 57-61; Figuren * | 1 | |
| A | US-A-4 362 681 (SPECTOR et al.) <br> * Figur 1 * | 1 | |

| | | | RECHERCHIERTE SACHGEBIETE (Int. Cl.4) |
|---|---|---|---|
| A | DE-B-2 620 907 (BATTELLE-INSTITUT EV.) | | A 61 F 2/00 <br> A 61 L 27/00 |
| A | DE-A-2 546 824 (E. LEITZ GMBH) | | |
| A | DE-A-2 659 591 (SUMITOMO CHEMICAL CO., LTD.) | | |
| A | DE-A-2 708 917 (R. BOSCH GMBH) | | |

--- -/-

Der vorliegende Recherchenbericht wurde für alle Patentansprüche erstellt.

| Recherchenort | Abschlußdatum der Recherche | Prüfer |
|---|---|---|
| BERLIN | 15-03-1985 | KANAL P K |

**KATEGORIE DER GENANNTEN DOKUMENTEN**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| A | US-A-3 918 100 (SHAW et al.) | | |

-----

RECHERCHIERTE SACHGEBIETE (Int Cl 4)

Der vorliegende Recherchenbericht wurde für alle Patentansprüche erstellt.

| Recherchenort | Abschlußdatum der Recherche | Prüfer |
|---|---|---|
| BERLIN | 15-03-1985 | KANAL P K |